# EUROPEAN PATENT APPLICATION

(11) **EP 3 184 020 A1**
(43) Date of publication of application: **28.06.2017**
(21) Application number: 15833574.5
(22) Date of filing: 27.04.2015
(51) Int. Cl.: A61B 1/00

(54) **CAPSULE MEDICAL DEVICE GUIDANCE SYSTEM**

(30) Priority: 20.08.2014 JP 2014167962
(71) Applicant: Olympus Corporation, Tokyo 192-8507 (JP)
(72) Inventor: CHIBA, Atsushi, Hachioji-shi Tokyo 192-8507 (JP); KAWANO, Hironao, Hachioji-shi Tokyo 192-8507 (JP)
(74) Representative: Gunzelmann, Rainer
(86) International application number: PCT/JP2015/062658
(87) International publication number: WO 2016/027517

(57) **Abstract**

Provided is a capsule medical device guide system that allows a user to easily recognize that a capsule medical device is separated from a boundary of a liquid when the capsule medical device is guided in the liquid introduced into a subject. The capsule medical device guide system 1 includes: a magnetic field generating unit 25 configured to generate a magnetic field that guides a capsule endoscope 10; a position detecting unit 22 configured to detect a position of the capsule endoscope 10 to output positional information; a display unit 23 configured to display the position of the capsule endoscope 10 within the subject; and a control unit 26 configured to cause, based on the positional information, the display unit 23 to display the position of the capsule endoscope 10 within the subject and a boundary position of a range in which the capsule endoscope 10 is capable of being guided within the subject.

## Description

### Field

The present invention relates to a capsule medical device guide system that guides a capsule medical device introduced into a subject.

### Background

Conventionally, a capsule medical device has been developed. The capsule medical device is introduced into a subject and obtains various kinds of information about the inside of the subject, or administers a medical agent or the like into the subject. For example, a capsule endoscope formed to have such a size as to allow itself to be introduced into a digestive tract of a subject is known.

The capsule endoscope includes an imaging function and a wireless communication function inside a capsule-shaped casing. The capsule endoscope is swallowed by a subject and performs capturing while moving through the inside of a digestive tract by means of peristaltic movement or the like. The capsule endoscope then sequentially and wirelessly transmits image data of images of the inside of an organ of the subject (hereinafter also referred to as an in-vivo image). The wirelessly transmitted image data are received by a receiving device provided outside the subject and obtained by an image display device such as a workstation to be subjected to a predetermined image process. Consequently, the in-vivo image of the subject can be displayed in the form of a still image or a motion image.

In recent years, a guide system that guides, by means of a magnetic field, a capsule endoscope introduced into a subject has been proposed. For example, Patent Literature 1 discloses a guide system that guides a capsule endoscope by means of a magnetic field. Specifically, a permanent magnet is provided inside the capsule endoscope, and a magnetic field generating unit such as an electromagnet and a permanent magnet is provided in a guide device. A liquid such as water is then introduced into a digestive tract (e.g., stomach) of a subject, and the capsule endoscope is caused to float in the liquid. In this state, the capsule endoscope is guided by a magnetic field generated by the magnetic field generation unit. The guide system is provided with a display device that receives image data obtained by the capsule endoscope and displays an in-vivo image. This allows a user to operate the guidance for the capsule endoscope using an operation input unit provided on the guide device with reference to the in-vivo image displayed on the display device.

### Citation List

### Patent Literature

Patent Literature 1: JP 2010-17554 A

### Summary

### Technical Problem

When a capsule endoscope is guided in a liquid, the capsule endoscope is temporarily brought into contact with a boundary of the liquid (e.g., a water surface or a water bottom), and a position and a posture are stabilized. After that, the capsule endoscope is separated from the boundary and caused to float in the liquid, and guide operation desired by a user is performed. In this case, whether the capsule endoscope is separated from the boundary, in particular, whether the capsule endoscope kept in contact with the water bottom is separated and floats from the water bottom, or whether the capsule endoscope kept in contact with the water surface is separated and sinks from the water surface, are determined in such a manner that an in-vivo image obtained by the capsule endoscope and displayed on a display device is observed by the user.

However, it is sometimes difficult to determine whether the capsule endoscope is separated from the water bottom or the water surface based only on the image. More specifically, in a case where a monocular capsule endoscope having an imaging unit provided only on one end side of a casing is used, it is difficult to determine whether the capsule endoscope floats from the water bottom when the imaging unit is directed to the water surface side. On the other hand, when the imaging unit is directed to the water bottom side, it is difficult to determine whether the capsule endoscope sinks from the water surface. Similarly, in a case where a compound eye capsule endoscope having imaging units provided on both ends of a casing is used, it is difficult to determine whether the capsule endoscope floats from the water bottom or sinks from the water surface when a user keeps a careful watch only on an image obtained by the imaging unit directed to the water surface. Even when the user keeps a careful watch on an image obtained by the opposite imaging unit, since the image only slightly changes when, for example, a movement amount of the capsule endoscope from the water bottom is small and a separation distance from the water bottom is very short, it is still difficult to determine whether the capsule endoscope floats from the water bottom or sinks from the water surface.

The present invention has been made in consideration of the above-mentioned problem, and an object thereof is to provide a capsule medical device guide system that allows a user to easily recognize that a capsule medical device is separated from a boundary of a liquid when the capsule medical device is guided in the liquid introduced into a subject.

### Solution to Problem

To solve the above-described problem and achieve the object, a capsule medical device guide system according to the present invention is configured to guide a capsule medical device that captures a subject within the subject into which a liquid is introduced, and includes: a guide unit configured to guide the capsule medical device; a position detecting unit configured to detect a position of the capsule medical device to output positional information; a display unit configured to display the position of the capsule medical device within the subject; and a control unit configured to cause, based on the positional information, the display unit to display the position of the capsule medical device within the subject and a boundary position of a range in which the capsule medical device is capable of being guided within the subject.

In the above-described capsule medical device guide system, the control unit sets a maximum value or a minimum value of the position of the capsule medical device as the boundary position.

The above-described capsule medical device guide system further includes an operation input unit configured to input, to the control unit, a signal that depends on operation from the outside, and the control unit resets the boundary position in accordance with the signal input from the operation input unit.

In the above-described capsule medical device guide system, either a first guide mode in which the capsule medical device is guided while in contact with a boundary of the area or a second guide mode in which the capsule medical device is guided while being separated from the boundary of the area and floating in the liquid is capable of being set switchably, and the control unit sets, as the boundary position, a maximum value or a minimum value of a position reached by the capsule medical device while the first guide mode is set.

In the above-described capsule medical device guide system, the first guide mode is a guide mode in which the capsule medical device is guided while in contact with a boundary of the area on a lower side in a vertical direction, and the control unit sets, as the boundary position, a minimum value of the position of the capsule medical device in the vertical direction in which an upward orientation is assumed to be positive.

In the above-described capsule medical device guide system, the first guide mode is a guide mode in which the capsule medical device is guided while in contact with a boundary of the area on an upper side in a vertical direction, and the control unit sets, as the boundary position, a maximum value of the position of the capsule medical device in the vertical direction in which an upward orientation is assumed to be positive.

The above-described capsule medical device guide system further includes an operation input unit configured to input, to the control unit, a signal that depends on operation from the outside, and the control unit sets, as the boundary position, the position of the capsule medical device at a point of time when predetermined operation input is performed on the operation input unit.

In the above-described capsule medical device guide system, the control unit resets the boundary position in accordance with the signal input from the operation input unit.

In the above-described capsule medical device guide system, either a first guide mode in which the capsule medical device is guided in such a direction as to bring the capsule medical device into contact with a boundary of the area or a second guide mode in which the capsule medical device is guided in such a direction as to cause the capsule medical device to be separated from the boundary of the area and float in the liquid is capable of being set switchably, and the control unit sets, as the boundary position, the position of the capsule medical device at a point of time when the first guide mode is switched to the second guide mode.

In the above-described capsule medical device guide system, the control unit is configured to: cause the display unit to display a scale representing the position of the capsule medical device in a predetermined direction within the subject, and to display, on the scale, a first indicator representing the boundary position and a second indicator representing a current position of the capsule medical device, when the first guide mode is set, cause positions of the first and second indicators to coincide, and cause the first and second indicators to move on the scale in accordance with the position of the capsule medical device, and when the second guide mode is set, fix the position of the first indicator to the scale, and cause only the second indicator to move on the scale in accordance with the position of the capsule medical device.

The above-described capsule medical device guide system further includes a determination unit configured to determine, based on a difference between the position of the capsule medical device and the boundary position, a contact state of the capsule medical device with respect to a boundary of the area, and the control unit causes the display unit to display a determination result produced by the determination unit.

### Advantageous Effects of Invention

According to the present invention, a position of a capsule medical device within a subject and a boundary position of a range in which the capsule medical device is capable of being guided within the subject are displayed on a display unit. Therefore, a user can easily recognize that the capsule medical device is separated from a boundary of a liquid.

### Brief Description of Drawings

FIG. 1 is a diagram illustrating an exemplary configuration of a capsule medical device guide system according to a first embodiment of the present invention.
FIG. 2 is a schematic diagram illustrating an exemplary internal structure of a capsule endoscope illustrated in FIG. 1.
FIG. 3 is a schematic diagram illustrating an exemplary configuration of an external appearance of a guide device illustrated in FIG. 1.
FIG. 4 is a diagram illustrating an exemplary operation input unit illustrated in FIG. 1.
FIG. 5 is a diagram for explaining guidance for the capsule endoscope that can be operated by the operation input unit illustrated in FIG. 1.
FIG. 6 is a schematic diagram illustrating an exemplary screen displayed on a display unit.
FIG. 7 is a schematic diagram for explaining an examination method using the capsule medical device guide system illustrated in FIG. 1.
FIG. 8 is a flowchart illustrating operation of the capsule medical device guide system according to the first embodiment of the present invention.
FIG. 9 is a schematic diagram illustrating a display form of a positional information display area that depends on guide operation for the capsule endoscope.
FIG. 10 is a schematic diagram illustrating a display form of the positional information display area that depends on the guide operation for the capsule endoscope.
FIG. 11 is a schematic diagram illustrating a display form of the positional information display area that depends on the guide operation for the capsule endoscope.
FIG. 12 is a schematic diagram illustrating a display form of the positional information display area that depends on the guide operation for the capsule endoscope.
FIG. 13 is a flowchart illustrating operation of a capsule medical device guide system according to a second embodiment of the present invention.
FIG. 14 is a schematic diagram illustrating an exemplary configuration of a capsule medical device guide system according to a first variation of the first and second embodiments of the present invention.
FIG. 15 is a schematic diagram illustrating an exemplary display of a determination result produced by a determination unit illustrated in FIG. 14.
FIG. 16 is a schematic diagram illustrating a display form of a positional information display area that depends on guide operation for a capsule endoscope in a fourth variation of the first and second embodiments of the present invention.

### Description of Embodiments

Hereinafter, a capsule medical device guide system according to an embodiment of the present invention will be described with reference to the drawings. In the following description, a capsule endoscope that is orally introduced into a subject to capture the inside of the subject (inside of a lumen) is described as an example of a capsule medical device that is guided in the capsule medical device guide system according to the present embodiment. However, the present invention is not limited by this embodiment. In other words, the present invention can be applied to guidance for various capsule-shaped medical devices such as, for example, a capsule endoscope that moves through, while capturing, the inside of a lumen ranging from an esophagus to an anus of a subject, a capsule medical device that delivers a medical agent or the like into a subject, and a capsule medical device including a pH sensor that measures pH within a subject.

In the following description, a shape, a size, and a positional relation are only schematically illustrated in each drawing to such an extent that contents of the present invention can be understood. Therefore, the present invention is not limited only to the shape, the size, and the positional relation represented in each drawing. In the drawings, identical elements are provided with the same reference signs.

### (First Embodiment)

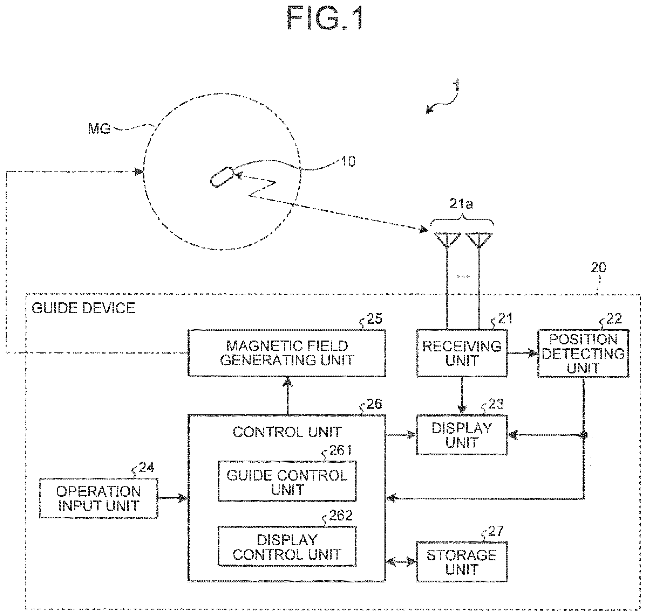

FIG. 1 is a diagram illustrating an exemplary configuration of a capsule medical device guide system according to a first embodiment of the present invention. As illustrated in FIG. 1, the capsule medical device guide system 1 according to the first embodiment includes a capsule endoscope 10 and a guide device 20. The capsule endoscope 10 is an example of a capsule medical device. The guide device 20 guides the capsule endoscope 10 introduced into a subject. The first embodiment employs such a method that a permanent magnet is provided inside the capsule endoscope 10, and a magnetic field MG is applied to the capsule endoscope 10, whereby the capsule endoscope 10 is guided.

The capsule endoscope 10 is introduced into an organ of a subject together with a predetermined liquid by means of oral ingestion or the like, moves through the inside of a digestive tract, and is eventually discharged to the outside of the subject. During this time, the capsule endoscope 10 floats in the liquid within the organ of the subject such as a stomach, and captures the inside of the subject to sequentially generate image data of in-vivo images while being guided by the magnetic field MG. The capsule endoscope 10 then wirelessly transmits the image data.

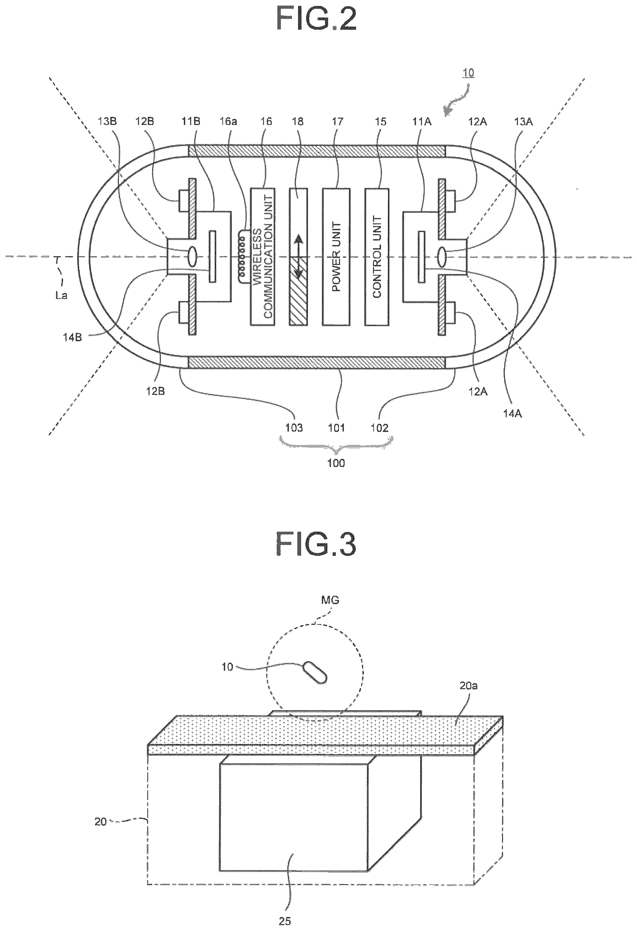

FIG. 2 is a schematic diagram illustrating an exemplary internal structure of the capsule endoscope 10. As illustrated in FIG. 2, the capsule endoscope 10 includes a capsule-shaped casing 100, imaging units 11A, 11B, a control unit 15, a wireless communication unit 16, a power unit 17, and a permanent magnet 18. The capsule-shaped casing 100 is an exterior formed to have such a size as to allow itself to be easily introduced into an organ of a subject. The imaging units 11A, 11B capture images of objects in imaging directions different from each other. The control unit 15 processes a signal input from each of the imaging units 11A, 11B and controls each component of the capsule endoscope 10. The wireless communication unit 16 wirelessly transmits the signal processed by the control unit 15 to the outside of the capsule endoscope 10. The power unit 17 supplies power to each component of the capsule endoscope 10. The permanent magnet 18 enables the guide device 20 to perform guidance.

The capsule-shaped casing 100 is an outer casing formed to have such a size as to allow itself to be introduced into an organ of a subject. The capsule-shaped casing 100 has a tubular casing 101 and dome-shaped casings 102, 103, and is configured such that both opening ends of the tubular casing 101 are closed by the dome-shaped casings 102, 103. The tubular casing 101 is a colored casing that is substantially opaque to visible light. On the other hand, each of the dome-shaped casings 102, 103 is a dome-like optical member that is transparent to light having a predetermined wavelength band such as visible light. As illustrated in FIG. 2, this capsule-shaped casing 100 liquid-tightly contains the imaging units 11A, 11B, the control unit 15, the wireless communication unit 16, the power unit 17, and the permanent magnet 18.

The imaging unit 11A has an illumination unit 12A such as an LED, an optical system 13A such as a condenser lens, and an image sensor 14A such as a CMOS image sensor or a CCD. The illumination unit 12A emits illumination light such as white light to an imaging field of the image sensor 14A and illuminates a subject within the imaging field through the dome-shaped casing 102. The optical system 13A collects reflected light from the imaging field at an imaging surface of the image sensor 14A to form an image. The image sensor 14A converts, into an electrical signal, the reflected light (optical signal) from the imaging field received at the imaging surface, and outputs the electrical signal as an image signal.

In the same way as the imaging unit 11A, the imaging unit 11B has an illumination unit 12B such as an LED, an optical system 13B such as a condenser lens, and an image sensor 14B such as a CMOS image sensor or a CCD. The imaging unit 11B captures a subject within an imaging field through the dome-shaped casing 103.

As illustrated in FIG. 2, in a case where the capsule endoscope 10 is a compound eye capsule medical device that captures both ends in a direction of a long axis La, that is, the front and the rear, the imaging units 11A, 11B are arranged so that an optical axis of each of them is substantially parallel to or substantially coincides with the long axis La that is a central axis in a longitudinal direction of the capsule-shaped casing 100, and the respective imaging fields face in opposite directions. In other words, the imaging units 11A, 11B are mounted so that the imaging surfaces of the image sensors 14A, 14B are orthogonal to the long axis La.

The control unit 15 controls operation of each of the imaging units 11A, 11B and operation of the wireless communication unit 16 and controls input and output of a signal between these components. More specifically, the control unit 15 causes the image sensor 14A to capture a subject within the imaging field illuminated with the illumination unit 12A, and causes the image sensor 14B to capture a subject within the imaging field illuminated with the illumination unit 12B. The control unit 15 then performs a predetermined signal process on an image signal output from each of the image sensors 14A, 14B. The control unit 15 further causes the wireless communication unit 16 to sequentially and wirelessly transmit the above-mentioned image signals in time series.

The wireless communication unit 16 includes an antenna 16a for transmitting a wireless signal. The wireless communication unit 16 obtains, from the control unit 15, an image signal of an in-vivo image generated by each of the imaging units 11A, 11B that have captured a subject, and performs a modulation process or the like on the image signal to generate a wireless signal. The wireless communication unit 16 then transmits the generated wireless signal via the antenna 16a.

The power unit 17 is a power storage unit such as a button type battery and a capacitor, and has a switch unit such as a magnetic switch and an optical switch. When the power unit 17 is configured to have the magnetic switch, an on/off state of a power source is switched by a magnetic field applied from the outside. In the on state, power in the power storage unit is appropriately supplied to each component of the capsule endoscope 10, that is, the imaging units 11A, 11B, the control unit 15, and the wireless communication unit 16. In the off state, the power unit 17 stops supplying power to each component of the capsule endoscope 10.

The permanent magnet 18 enables the magnetic field MG generated by a magnetic field generating unit 25 to guide the capsule endoscope 10. The permanent magnet 18 is arranged to be fixed within the capsule-shaped casing 100 so that a magnetization direction is inclined to the long axis La. In FIG. 2, the magnetization direction of the permanent magnet 18 is represented by an arrow. In the first embodiment, the permanent magnet 18 is arranged so that the magnetization direction is orthogonal to the long axis La. The permanent magnet 18 operates in accordance with a magnetic field applied from the outside. As a result, guidance for the capsule endoscope 10 by the magnetic field generating unit 25 is realized.

Referring again to FIG. 1, the guide device 20 includes a receiving unit 21, a position detecting unit 22, a display unit 23, an operation input unit 24, the magnetic field generating unit 25, a control unit 26, and a storage unit 27. The receiving unit 21 wirelessly communicates with the capsule endoscope 10 to receive a wireless signal transmitted from the capsule endoscope 10. The position detecting unit 22 detects a position of the capsule endoscope 10 within a subject based on the wireless signal received by the receiving unit 21. The display unit 23 obtains an image signal from the wireless signal received by the receiving unit 21, performs a predetermined signal process on the image signal to display an in-vivo image on a screen, and displays the position of the capsule endoscope 10 within the subject on the screen. The operation input unit 24 accepts input of information or the like including an instruction for various types of operation in the capsule medical device guide system 1. The magnetic field generating unit 25 generates a magnetic field for guiding the capsule endoscope 10. The control unit 26 controls each of these components. The storage unit 27 stores image data or the like of an in-vivo image obtained by the capsule endoscope 10.

FIG. 3 is a schematic diagram illustrating an exemplary external appearance of the guide device 20 illustrated in FIG. 1. As illustrated in FIG. 3, the guide device 20 is provided with a bed 20a as a table on which a subject is placed. Below the bed 20a, at least the magnetic field generating unit 25 that generates the magnetic field MG is arranged.

The receiving unit 21 includes a plurality of receiving antennas 21a and sequentially receives wireless signals from the capsule endoscope 10 via these receiving antennas 21a. The receiving unit 21 selects an antenna having the highest reception electric field intensity from among these receiving antennas 21a, and performs a demodulation process or the like on a wireless signal from the capsule endoscope 10 received via the selected antenna. The receiving unit 21 thus extracts an image signal from the wireless signal and outputs the image signal to the display unit 23.

The position detecting unit 22 detects the position of the capsule endoscope 10 within a subject based on intensity of a wireless signal received by the receiving unit 21, and generates and outputs information about the position of the capsule endoscope 10. Hereinafter, the information about the position of the capsule endoscope 10 is simply referred to as positional information. For instance, as disclosed in JP 2007-283001 A, the position of the capsule endoscope 10 can be obtained in such a manner that an initial value of the position is appropriately set, and a process of calculating an estimate value of the position by means of the Gauss-Newton method is repeated until a shift amount between the calculated estimate value and a previous estimate value becomes equal to or less than a predetermined value. Alternatively, the position of the capsule endoscope 10 may be calculated in such a manner that a coil that generates a high-frequency magnetic field is provided in the capsule endoscope 10, and the magnetic field generated by this coil is detected outside a subject.

The display unit 23 has various displays such as a liquid crystal display. The display unit 23 displays an in-vivo image that is based on an image signal input from the receiving unit 21, positional information of the capsule endoscope 10, and various other types of information.

The operation input unit 24 accepts input of various types of information such as guide instruction information for guiding the capsule endoscope 10 and setting information for setting various modes for the guide device 20. The guide instruction information is information for controlling the position and a posture of the capsule endoscope 10 to be subjected to guide operation. The guide instruction information specifically includes information about translation operation, tilt angle changing operation, and azimuth angle changing operation or the like. The translation operation translates the capsule endoscope 10 in a horizontal direction or a vertical direction. The tilt angle changing operation changes a tilt angle of the long axis La of the capsule endoscope 10 with respect to the vertical direction. The azimuth angle changing operation changes an azimuth angle that is an angle around a vertical axis of a field of the capsule endoscope 10, i.e., respective fields of the imaging units 11A, 11B which will be described later. The operation input unit 24 inputs, to the control unit 26, these items of information that have been input and accepted.

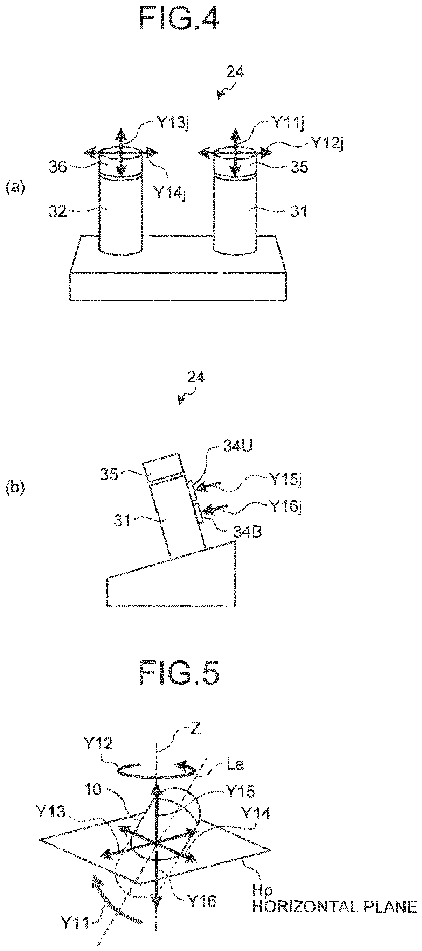

The operation input unit 24 is realized by, for example, a joystick, a console including various buttons and various switches, and an input device such as a keyboard. In the first embodiment, joysticks illustrated in FIG. 4 constitute the operation input unit 24. A front view of the operation input unit 24 is illustrated in (a) of FIG. 4, and a right side view of the operation input unit 24 is illustrated in (b) of FIG. 4. FIG. 5 is a diagram illustrating a movement of the capsule endoscope 10 instructed by operation for each component of the operation input unit 24.

As illustrated in (a) of FIG. 4, the operation input unit 24 includes two joysticks 31, 32 for three-dimensionally operating the guidance for the capsule endoscope 10 by the magnetic field generating unit 25. Tilting operation can be performed on each of the joysticks 31, 32 in up and down directions and left and right directions.

As illustrated in (b) of FIG. 4, a back surface of the joystick 31 is provided with an up button 34U and a down button 34B. The up button 34U is pressed to input, to the control unit 26, guide instruction information including an instruction for upward guidance for the capsule endoscope 10. The down button 34B is pressed to input, to the control unit 26, guide instruction information including an instruction for downward guidance for the capsule endoscope 10. A capture button 35 is provided on an upper part of the joystick 31. The capture button 35 is pressed to capture an in-vivo image displayed on the display unit 23. An approach button 36 is provided on an upper part of the joystick 32. The approach button 36 is pressed to input, to the control unit 26, guide instruction information for guiding the capsule endoscope 10 so that the imaging unit 11A of the capsule endoscope 10 is brought close to an object to be captured by the imaging unit 11A.

As illustrated in (a) of FIG. 4, a tilting direction of the joystick 31 in the up and down directions represented by an arrow Y11j corresponds to a tilting guide direction in which a distal end of the capsule endoscope 10 sways so as to pass through a vertical axis (Z axis) as represented by an arrow Y11 in FIG. 5. When guide instruction information corresponding to tilting operation of the arrow Y11j for the joystick 31 is input from the operation input unit 24 to the control unit 26, the control unit 26 (guide control unit 261 to be described later) calculates, based on this guide instruction information, a guide direction of the distal end of the capsule endoscope 10 on an absolute coordinate system in association with the tilting direction of the joystick 31, and calculates a guide amount in association with the tilting operation for the joystick 31. The control unit 26 changes a magnetic field generated by the magnetic field generating unit 25 so that an elevation angle of the capsule endoscope 10 is changed in the calculated guide direction in accordance with the calculated guide amount.

As illustrated in (a) of FIG. 4, a tilting direction of the joystick 31 in the left and right directions represented by an arrow Y12j corresponds to a rotation guide direction in which the capsule endoscope 10 rotates around the Z axis as represented by an arrow Y12 in FIG. 5. When guide instruction information corresponding to tilting operation of the arrow Y12j for the joystick 31 is input from the operation input unit 24 to the control unit 26, the control unit 26 calculates, based on this guide instruction information, a guide direction of the distal end of the capsule endoscope 10 on the absolute coordinate system in association with the tilting direction of the joystick 31, and calculates a guide amount in association with the tilting operation for the joystick 31. The control unit 26 changes a magnetic field generated by the magnetic field generating unit 25 so that the capsule endoscope 10 turns in the calculated guide direction in accordance with the calculated guide amount.

As illustrated in (a) of FIG. 4, a tilting direction of the joystick 32 in the up and down directions represented by an arrow Y13j corresponds to a horizontal backward guide direction or a horizontal forward guide direction in which the capsule endoscope 10 advances in a direction of the long axis La of the capsule endoscope 10 projected on a horizontal plane Hp as represented by an arrow Y13 in FIG. 5. When guide instruction information corresponding to tilting operation of the arrow Y13j for the joystick 32 is input from the operation input unit 24 to the control unit 26, the control unit 26 calculates, based on this guide instruction information, a guide direction and a guide amount of the distal end of the capsule endoscope 10 on the absolute coordinate system in association with the tilting direction of the joystick 32. The control unit 26 changes a magnetic field generated by the magnetic field generating unit 25 so that the capsule endoscope 10 is translated in accordance with the calculated guide direction and guide amount.

As illustrated in (a) of FIG. 4, a tilting direction of the joystick 32 in the left and right directions represented by an arrow Y14j corresponds to a horizontal right guide direction or a horizontal left guide direction in which the capsule endoscope 10 advances on the horizontal plane Hp in a direction vertical to the direction of the long axis La projected on the horizontal plane Hp as represented by an arrow Y14 in FIG. 5. When guide instruction information corresponding to tilting operation of the arrow Y14j for the joystick 32 is input from the operation input unit 24 to the control unit 26, the control unit 26 calculates, based on this guide instruction information, a guide direction and a guide amount of the distal end of the capsule endoscope 10 on the absolute coordinate system in association with the tilting direction of the joystick 32. The control unit 26 changes a magnetic field generated by the magnetic field generating unit 25 so that the capsule endoscope 10 is translated in accordance with the calculated guide direction and guide amount.

The back surface of the joystick 31 is provided with the up button 34U and the down button 34B. When the up button 34U is pressed as represented by an arrow Y15j in (b) of FIG. 4, an instruction for up operation is given by which the capsule endoscope 10 advances upward along the Z axis illustrated in FIG. 5 as represented by an arrow Y15. When the down button 34B is pressed as represented by an arrow Y16j in (b) of FIG. 4, an instruction for down operation is given by which the capsule endoscope 10 advances downward along the Z axis illustrated in FIG. 5 as represented by an arrow Y16.

When guide instruction information corresponding to pressing operation of each of the arrows Y15j, Y16j for the up button 34U and the down button 34B is input from the operation input unit 24 to the control unit 26, the control unit 26 calculates, based on this guide instruction information, a guide direction and a guide amount of the distal end of the capsule endoscope 10 on the absolute coordinate system in association with the pressed button. The control unit 26 changes a magnetic field generated by the magnetic field generating unit 25 so that the capsule endoscope 10 is translated in the vertical direction in accordance with the calculated guide direction and guide amount. For example, when the up button 34U is pressed, the magnetic field generating unit 25 weakens intensity of the magnetic field MG and reduces magnetic attracting force that acts on the permanent magnet 18 provided in the capsule endoscope 10. Consequently, the capsule endoscope 10 is elevated as represented by the arrow Y15. On the other hand, when the down button 34B is pressed, the magnetic field generating unit 25 strengthens intensity of the magnetic field MG and increases magnetic attracting force that acts on the permanent magnet 18 provided in the capsule endoscope 10. Consequently, the capsule endoscope 10 is lowered as represented by the arrow Y16.

In addition to these joysticks 31, 32, the operation input unit 24 may further have an input device including various operation buttons and a keyboard or the like.

The magnetic field generating unit 25 generates a magnetic field for changing, relative to a subject, the position, the tilt angle, and the azimuth angle of the capsule endoscope 10 introduced into the subject. A configuration of the magnetic field generating unit 25 is not particularly limited as long as the magnetic field MG, a magnetic gradient of which can be controlled by the control unit 26, can be formed in an area on the bed 20a on which a subject is placed. More specifically, the magnetic field generating unit 25 may include an electromagnet, or the magnetic field generating unit 25 may include a permanent magnet and a drive unit that changes a position or a direction of the permanent magnet.

The control unit 26 includes the guide control unit 261 and a display control unit 262. The guide control unit 261 controls operation of the magnetic field generating unit 25. The display control unit 262 controls a display form of an image and various types of information displayed on the display unit 23.

The guide control unit 261 controls the magnetic field generating unit 25 in a guide mode that depends on a signal input from the operation input unit 24 based on positional information of the capsule endoscope 10 input from the position detecting unit 22 and guide instruction information input from the operation input unit 24. The capsule endoscope 10 is thus guided to a position desired by a user. The guide mode includes a water bottom mode, an underwater mode, and a water surface mode. In the water bottom mode, the capsule endoscope 10 is guided while in contact with a bottom of a liquid introduced into an organ of a subject. In the underwater mode, the capsule endoscope 10 is guided while floating in a liquid. In the water surface mode, the capsule endoscope 10 is guided while floating on a surface of a liquid. Hereinafter, a surface, the inside, and a bottom of a liquid introduced into a subject are described as a water surface, underwater, and a water bottom, respectively. The liquid as used herein also includes a liquid other than water (e.g., saline or the like).

The display control unit 262 generates a screen with a predetermined format including an in-vivo image that is based on an image signal received by the receiving unit 21, positional information of the capsule endoscope 10, and various other types of information. The display control unit 262 causes the display unit 23 to display the screen.

The storage unit 27 includes a storage medium and a writing reading device. The storage medium, such as a flash memory or a hard disk, saves information in a rewritable manner. The writing reading device writes and reads information to and from the storage medium. The storage unit 27 stores image data of an in-vivo image group of a subject that is based on an image signal transmitted from the capsule endoscope 10. The storage unit 27 also stores information such as various programs and various parameters that is used by the control unit 26 to control each component of the guide device 20.

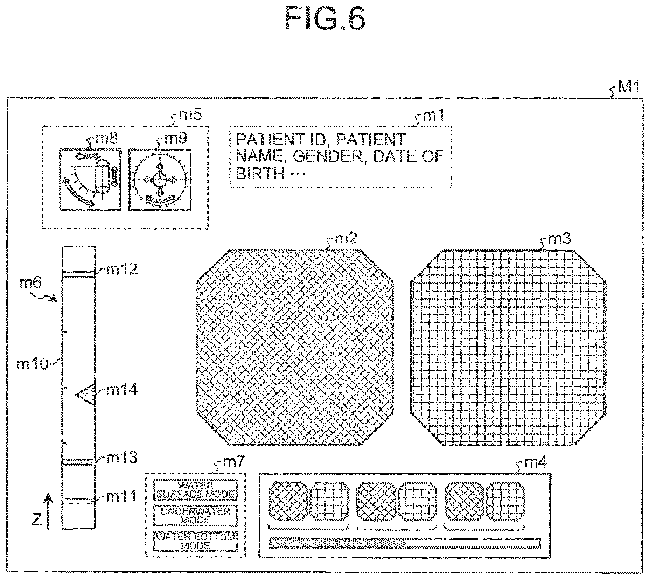

FIG. 6 is a schematic diagram illustrating an exemplary screen displayed on the display unit 23. A screen M1 illustrated in FIG. 6 includes a patient information display area m1, two in-vivo image display areas m2, m3, a capture image display area m4, an operation information display area m5, a positional information display area m6, and a guide mode display area m7. On the patient information display area m1, patient information such as a patient ID, a patient name, gender of a patient, and a date of birth is displayed. On the in-vivo image display areas m2, m3, in-vivo images obtained by the imaging units 11A, 11B are respectively displayed. On the capture image display area m4, a plurality of in-vivo images captured by predetermined operation for the operation input unit 24 is displayed. On the operation information display area m5, operation information for the capsule endoscope 10 is displayed. On the positional information display area m6, the position of the capsule endoscope 10 in the vertical direction (Z direction) within a subject is displayed. On the guide mode display area m7, a guide mode currently set in the guide device 20, namely, any of the water bottom mode, the underwater mode, and the water surface mode, is displayed.

The operation information display area m5 is an area on which a posture diagram m8 and a posture diagram m9 are displayed. The posture diagram m8 indicates the posture of the capsule endoscope 10 on a vertical plane, and the posture diagram m9 indicates the posture of the capsule endoscope 10 on a horizontal plane. On each of the posture diagrams m8, m9, a plurality of directions in which the capsule endoscope 10 can be guided is indicated by arrows. When there is operation input for guiding the capsule endoscope 10 in any of the directions, a display color of an arrow corresponding to the input direction among these arrows is changed. This assists a user to perform the guide operation.

Guide instruction information input from the operation input unit 24 is reflected in a control signal that is output when the guide control unit 261 controls the magnetic field generating unit 25. Therefore, the posture of the capsule endoscope 10 displayed on each of the posture diagrams m8, m9 can be considered to be substantially the same as the posture of the actual capsule endoscope 10 in a subject.

The positional information display area m6 includes a scale m10 and various marks. The scale m10 indicates the position in the Z direction. The various marks are displayed on the scale m10. More specifically, a working area lower limit mark m11, a working area upper limit mark m12, a water bottom mark m13, and a capsule current position mark m14 are displayed on the scale m10. The working area lower limit mark m11 indicates a lower limit position of an area in which the magnetic field MG capable of guiding the capsule endoscope 10 exists, that is, a working area. The working area upper limit mark m12 indicates an upper limit position of the working area. The water bottom mark m13 serves as a first indicator indicating a position of a bottom of a liquid introduced into an organ of a subject. The capsule current position mark m14 serves as a second indicator indicating a current position of the capsule endoscope 10.

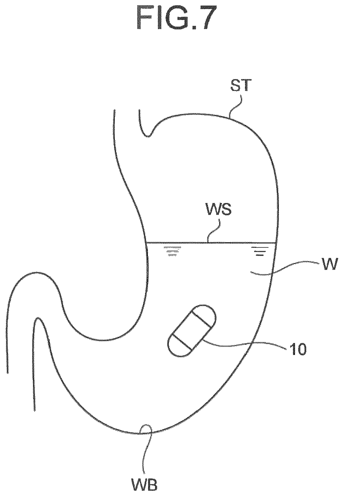

Next, an examination method using the capsule medical device guide system 1 will be described. FIG. 7 is a schematic diagram for explaining an examination method using the capsule medical device guide system 1. In the capsule medical device guide system 1, as illustrated in FIG. 7, observation is performed while the capsule endoscope 10 floats in a liquid W introduced into an organ ST of a subject by means of oral ingestion or the like. The liquid W is a liquid harmless to a human body such as, for example, water and saline. When such an examination is performed, a boundary of a range in which the capsule endoscope 10 can be guided within the subject is a water surface WS that is a surface of the liquid W, a water bottom WB that is a bottom surface of the liquid W, and an inner wall surface of a part of the organ ST filled with the liquid W.

In the first embodiment, the capsule endoscope 10 is designed so as to have a specific gravity less than a specific gravity of the liquid W and float on the liquid W while the guidance by the magnetic field generating unit 25 is not performed. In this case, owing to a balance between buoyancy of the capsule endoscope 10 with respect to the liquid W, gravity that acts on the capsule endoscope 10, and magnetic attracting force applied by the magnetic field generating unit 25, the capsule endoscope 10 can be stopped at a desired position within the liquid W to allow the inside of the organ ST to be observed.

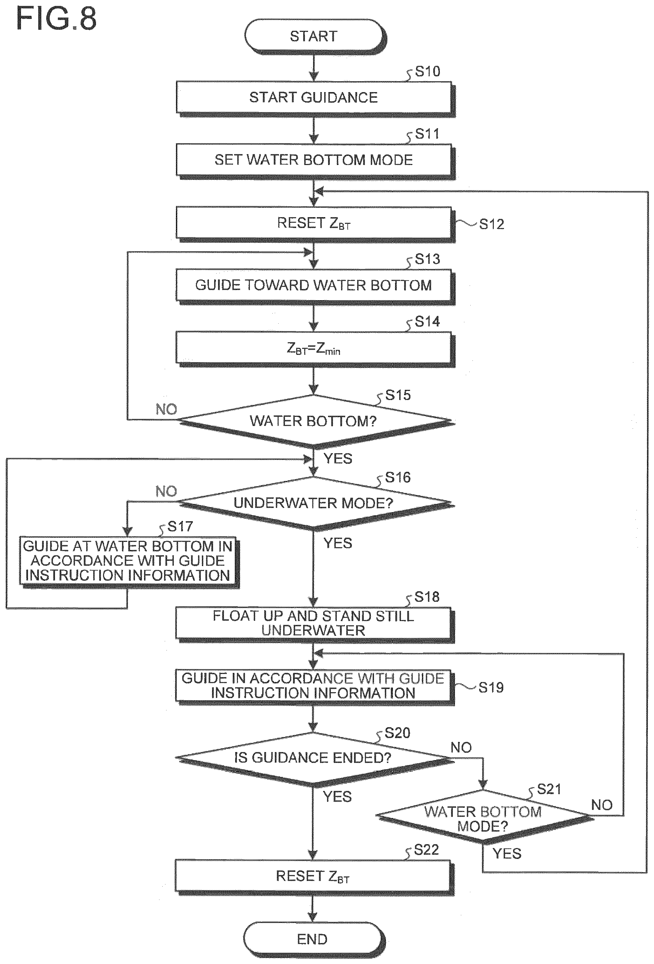

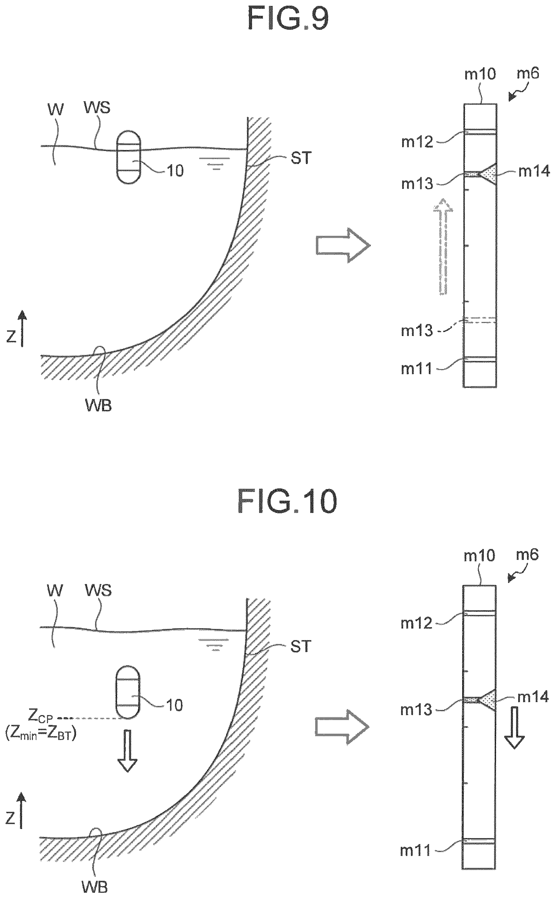

FIG. 8 is a flowchart illustrating operation of the capsule medical device guide system according to the first embodiment. FIGS. 9 to 12 are schematic diagrams illustrating states of the capsule endoscope 10 introduced into the organ ST and display forms of the positional information display area m6 that depend on guide operation for the capsule endoscope 10. Hereinafter, an upward orientation in the vertical direction (Z direction) is assumed to be positive.

When the subject is examined by the capsule medical device guide system 1, as illustrated in FIG. 7, the liquid W is introduced into the subject in advance, the power source of the capsule endoscope 10 is turned on, and the capsule endoscope 10 is swallowed by the subject.

In step S10, when a signal including an instruction to start guiding the capsule endoscope 10 is input from the operation input unit 24 to the control unit 26, each component of the guide device 20 starts operating. More specifically, the receiving unit 21 starts the operation to receive a wireless signal transmitted from the capsule endoscope 10, extract an image signal from the wireless signal, and output the image signal to the display unit 23. The position detecting unit 22 starts the operation to detect the position of the capsule endoscope 10 and output positional information. The display unit 23 starts, as illustrated in FIG. 6, displaying an in-vivo image that is based on the image signal output from the receiving unit 21.

The control unit 26 starts obtaining the positional information of the capsule endoscope 10 output from the position detecting unit 22, and starts the control to cause the display unit 23 to display the position of the capsule endoscope 10. More specifically, in the positional information display area m6 illustrated in FIG. 6, the capsule current position mark m14 is displayed at a position on the scale m10 corresponding to a coordinate value Z_{CP} that is a current Z coordinate of the capsule endoscope 10, and the capsule current position mark m14 is slid on the scale m10 in conjunction with the coordinate value Z_{CP}.

In the subsequent step S11, the control unit 26 sets the guide mode of the capsule endoscope 10 to the water bottom mode in which the guidance is performed while a part of the capsule endoscope 10 is in contact with the water bottom WB.

In step S12, the control unit 26 resets a coordinate value Z_{BT} in the Z direction indicating a position of the water bottom WB. Accordingly, as illustrated in FIG. 9, the display control unit 262 moves the water bottom mark m13 to a position currently indicated by the capsule current position mark m14.

In the subsequent step S13, the guide control unit 261 performs the control to guide the capsule endoscope 10 toward the water bottom WB. Specifically, the guide control unit 261 causes the magnetic field generating unit 25 to generate such a magnetic field that magnetic attracting force applied to the permanent magnet 18 provided inside the capsule endoscope 10 is strengthened.

In step S14, the control unit 26 sets, as the coordinate value Z_{BT} of the water bottom WB, a minimum value Zₘᵢₙ reached by the coordinate value Z_{CP} of the capsule endoscope 10 after the coordinate value Z_{BT} is reset in step S12. Consequently, as illustrated in FIG. 10, the minimum value Zₘᵢₙ of the coordinate value Z_{CP} of the capsule endoscope 10 is sequentially updated as the capsule endoscope 10 moves toward the water bottom WB. Therefore, the coordinate value Z_{BT} of the water bottom WB is also sequentially updated in accordance with the minimum value Zₘᵢₙ. Correspondingly, in the positional information display area m6, the water bottom mark m13 slides in accordance with the coordinate value Z_{BT}. While the capsule endoscope 10 moves toward the water bottom in the water bottom mode, therefore, the capsule current position mark m14 and the water bottom mark m13 slide downward in the overlapping state.

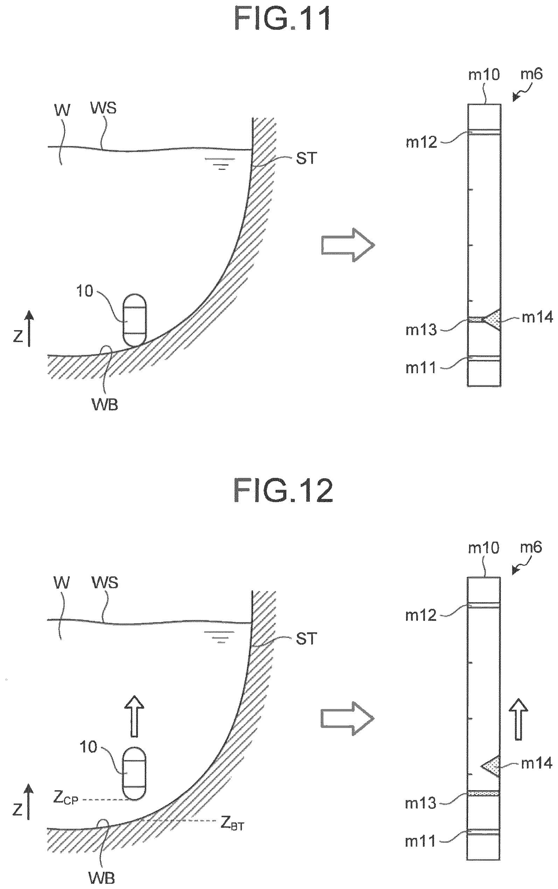

In step S15, the control unit 26 determines whether the capsule endoscope 10 reaches the water bottom WB. When the coordinate value Z_{CP} of the capsule endoscope 10 does not change even though a magnetic field that guides the capsule endoscope 10 in a -Z direction is generated by the magnetic field generating unit 25, the capsule endoscope 10 is determined to reach the water bottom WB as illustrated in FIG. 11.

When the capsule endoscope 10 does not reach the water bottom WB (step S15: No), the operation of the guide device 20 returns to step S13. On the other hand, when the capsule endoscope 10 reaches the water bottom WB (step S15: Yes), the control unit 26 then determines whether a signal designating the underwater mode is input from the operation input unit 24 (step S16).

When the signal designating the underwater mode is not input (step S16: No), the guide control unit 261 performs, in accordance with guide instruction information input from the operation input unit 24, the control to guide the capsule endoscope 10 at the water bottom WB while feeding back positional information of the capsule endoscope 10 (step S17). More specifically, the tilt angle and the azimuth angle of the capsule endoscope 10 are changed while a part of the capsule endoscope 10 is in contact with the water bottom WB.

On the other hand, when the signal designating the underwater mode is input (step S16: Yes), the guide control unit 261 performs the guide control to cause the capsule endoscope 10 to float from the water bottom WB and stand still underwater (step S18). More specifically, magnetic attracting force for the capsule endoscope 10 is temporarily weakened to cause the capsule endoscope 10 to float from the water bottom WB, and the magnetic attracting force is adjusted so that the gravity, the buoyancy, and the magnetic attracting force that act on the capsule endoscope 10 are balanced.

After this guide control causes the capsule endoscope 10 to float from the water bottom WB, as illustrated in FIG. 12, in the positional information display area m6, the capsule current position mark m14 slides upward in accordance with the coordinate value Z_{CP} of the capsule endoscope 10. On the other hand, since the coordinate value Z_{BT} of the water bottom does not change and remains at the minimum value Zₘᵢₙ, the position of the water bottom mark m13 also does not change.

At this time, a user visually recognizes that the capsule current position mark m14 is separated from the water bottom mark m13, whereby the user can easily grasp that the capsule endoscope 10 has floated from the water bottom WB. Therefore, the guide operation for the capsule endoscope 10 using the operation input unit 24 can be started after it is confirmed that the capsule endoscope 10 has floated from the water bottom WB and stood still underwater.

In the subsequent step S19, the guide control unit 261 performs, in accordance with guide instruction information input from the operation input unit 24, the control to guide the capsule endoscope 10 underwater while feeding back positional information of the capsule endoscope 10. After the guidance in the underwater mode is started, if the minimum value Zₘᵢₙ of the coordinate value Z_{CP} of the capsule endoscope 10 is updated, the coordinate value Z_{BT} of the water bottom may be or may not be updated in accordance with the minimum value Zₘᵢₙ. For example, in a case where the inner wall of the organ ST is inclined, and the capsule endoscope 10 moves to a deeper position, the minimum value Zₘᵢₙ of the coordinate value Z_{CP} can be updated.

In the subsequent step S20, the control unit 26 determines whether a signal including an instruction to end the guidance for the capsule endoscope 10 is input from the operation input unit 24.

When the signal including the instruction to end the guidance is not input (step S20: No), the control unit 26 then determines whether a signal designating the water bottom mode is input from the operation input unit 24 (step S21). The water bottom mode is selected, for example, when the guidance for the capsule endoscope 10 is performed all over again underwater. When the signal designating the water bottom mode is input (step S21: Yes), the operation of the guide device 20 returns to step S12. On the other hand, when the signal designating the water bottom mode is not input (step S21: No), the operation of the guide device 20 returns to step S19.

In step S20, when the signal including the instruction to end the guidance is input (step S20: Yes), the control unit 26 resets the coordinate value Z_{BT} of the water bottom WB (step S22). After that, the operation of the guide device 20 is ended.

As described above, according to the first embodiment, both the water bottom mark m13 indicating the position of the water bottom WB and the capsule current position mark m14 indicating the current position of the capsule endoscope 10 are simultaneously displayed on the positional information display area m6. Therefore, the user can easily recognize that the capsule endoscope 10 is separated from the water bottom WB by observing the positional information display area m6.

In addition, according to the first embodiment, the minimum value Zₘᵢₙ of the coordinate value Z_{CP} of the capsule endoscope 10 is set as the coordinate value Z_{BT} of the water bottom. Therefore, the coordinate value Z_{BT} of the water bottom can be accurately obtained.

### (Second Embodiment)

Next, a second embodiment of the present invention will be described.

A configuration of a capsule medical device guide system according to the second embodiment is similar to that of the first embodiment. A method for determining the coordinate value Z_{BT} of the water bottom WB is different from that of the first embodiment.

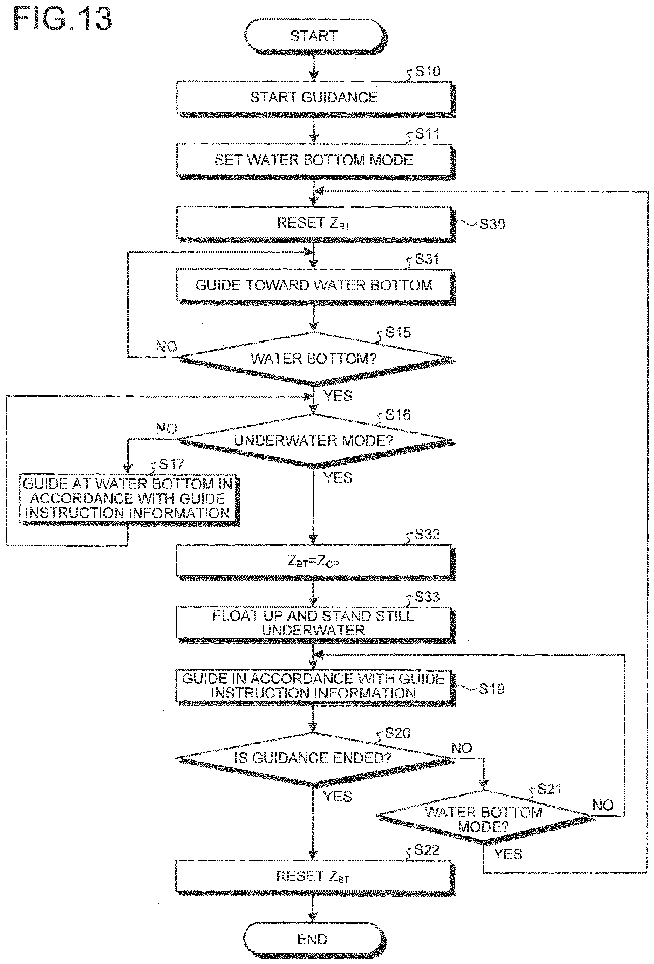

FIG. 13 is a flowchart illustrating operation of the capsule medical device guide system according to the second embodiment. In the flowchart, operation of step S10 and operation of step S11 are similar to those of the first embodiment.

In step S30 subsequent to step S11, the control unit 26 resets the coordinate value Z_{BT} in the Z direction indicating the position of the water bottom WB. Accordingly, as illustrated in FIG. 9, the display control unit 262 moves the water bottom mark m13 to a position currently indicated by the capsule current position mark m14.

In step S31, the guide control unit 261 performs the control to guide the capsule endoscope 10 toward the water bottom WB. The subsequent steps S15 to S17 are similar to those of the first embodiment.

In step S16, when the signal designating the underwater mode is input from the operation input unit 24 to the control unit 26 (step S16: Yes), the control unit 26 sets a current coordinate value Z_{CP} of the capsule endoscope 10 as the coordinate value Z_{BT} of the water bottom WB (step S32). At this time, as illustrated in FIG. 11, in the positional information display area m6, the water bottom mark m13 and the capsule current position mark m14 overlap each other and stop.

In the subsequent step S33, the guide control unit 261 performs the guide control to cause the capsule endoscope 10 to float from the water bottom WB and stand still underwater. After this guide control causes the capsule endoscope 10 to float from the water bottom, in the display unit 23, as illustrated in FIG. 12, only the capsule current position mark m14 slides upward in accordance with the coordinate value Z_{CP} of the capsule endoscope 10. On the other hand, since the coordinate value Z_{BT} of the water bottom is maintained at the coordinate value Z_{CP} of the capsule endoscope 10 at a point of time when the underwater mode is set, the position of the water bottom mark m13 also does not change. The subsequent steps S19 to S22 are similar to those of the first embodiment.

As described above, according to the second embodiment, the coordinate value Z_{CP} of the capsule endoscope 10 obtained when the water bottom mode is changed to the underwater mode is set as the coordinate value Z_{BT} of the water bottom WB. Therefore, the coordinate value Z_{BT} of the water bottom WB does not need to be updated in accordance with the change of the coordinate value Z_{CP} of the capsule endoscope 10, and the process can be simplified.

### (First Variation)

Next, a first variation of the above-mentioned first and second embodiments will be described.

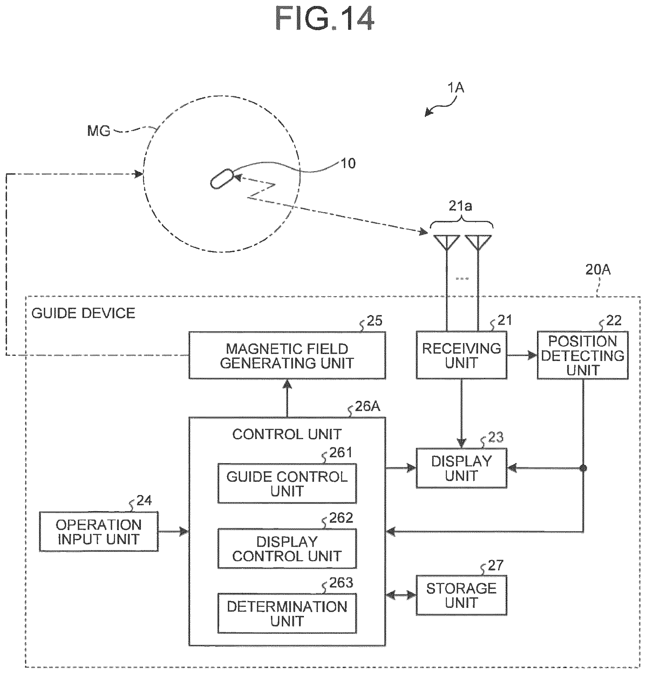

FIG. 14 is a schematic diagram illustrating an exemplary configuration of a capsule medical device guide system according to the first variation. As illustrated in FIG. 14, a capsule medical device guide system 1A according to the first variation includes the capsule endoscope 10 and a guide device 20A that guides the capsule endoscope 10. The guide device 20A includes a control unit 26A in place of the control unit 26 illustrated in FIG. 1. A configuration and operation of the capsule endoscope 10 and a configuration and operation of each component of the guide device 20A of the control unit 26A are similar to those of the first embodiment.

The control unit 26A further includes a determination unit 263 in addition to the guide control unit 261 and the display control unit 262. Operation of the guide control unit 261 and operation of the display control unit 262 are similar to those of the first embodiment.

The determination unit 263 determines, based on positional information of the capsule endoscope 10 output from the position detecting unit 22, a contact state of the capsule endoscope 10 with respect to a boundary surface of the liquid W introduced into the subject as illustrated in FIG. 7. Specifically, the determination unit 263 determines whether the capsule endoscope 10 is in contact with the water surface WS, the water bottom WB, or the inner wall surface of a part of the organ ST filled with the liquid W. More specifically, the determination unit 263 calculates a difference ΔZ between the coordinate value Z_{CP} of the capsule endoscope 10 and the coordinate value Z_{BT} of the water bottom when the capsule endoscope 10 is caused to float from the water bottom in step S18 in FIG. 8. When the difference ΔZ is greater than a threshold value, the capsule endoscope 10 is determined to be separated from the water bottom.

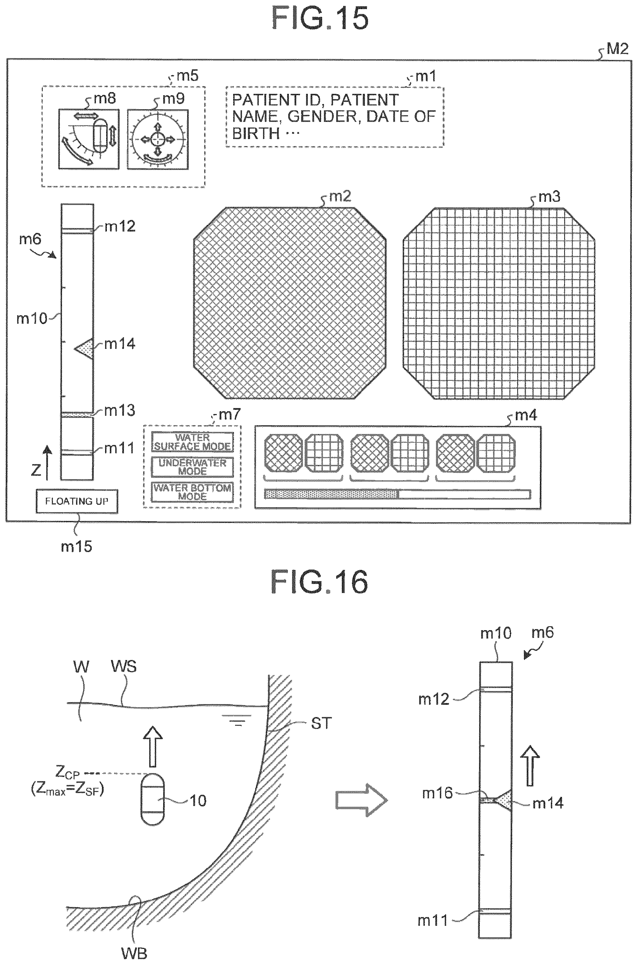

When the determination unit 263 determines that the capsule endoscope 10 is separated from the water bottom WB, the display control unit 262 causes the display unit 23 to display, as a determination result, a notification indicating that the capsule endoscope 10 has floated from the water bottom WB. For example, as illustrated in FIG. 15, text "floating up" may be displayed on an area m15 in the vicinity of the scale m10 of a screen M2.

As described above, according to the first variation, a user can more easily recognize that the capsule endoscope 10 is separated from the water bottom WB within the subject.

### (Second Variation)

Next, a second variation of the above-mentioned first and second embodiments will be described.

In the above-mentioned first and second embodiments, when the guidance for the capsule endoscope 10 is started, the guide mode is automatically set (refer to steps S10 and S11 in FIGS. 8 and 13). However, the water bottom mode may be set when a signal designating the water bottom mode is input from the operation input unit 24 to the control unit 26 in accordance with operation by a user for the operation input unit 24.

### (Third Variation)

Next, a third variation of the above-mentioned first and second embodiments will be described.

In the above-mentioned first and second embodiments, when the guide mode is set to the water bottom mode, the capsule endoscope 10 is guided to the water bottom WB by means of the automatic control performed by the guide control unit 261. However, the capsule endoscope 10 may be guided to the water bottom WB by means of manual operation by a user for the operation input unit 24. In this case, the guide control unit 261 performs, in accordance with guide instruction information input from the operation input unit 24, the control to guide the capsule endoscope 10 to the water bottom WB while feeding back positional information of the capsule endoscope 10.

In this case, in the same way as the first embodiment, the coordinate value Z_{BT} of the water bottom WB may be updated along with the minimum value Zₘᵢₙ of the coordinate value Z_{CP} of the capsule endoscope 10 as illustrated in FIG. 10. Alternatively, a current coordinate value Z_{CP} of the capsule endoscope 10 may be set as the coordinate value Z_{BT} of the water bottom WB by predetermined operation for the operation input unit 24.

### (Fourth Variation)

Next, a fourth variation of the above-mentioned first and second embodiments will be described.

The above-mentioned first and second embodiments have described the guide control for the capsule endoscope 10 performed when the water bottom mode is changed to the underwater mode. However, similar control may be performed when the water surface mode is changed to the underwater mode.

More specifically, the capsule endoscope 10 is designed so as to have a specific gravity greater than the specific gravity of the liquid W and sink in the liquid W while the guidance by the magnetic field generating unit 25 is not performed.

In the guide device 20, after the guidance for the capsule endoscope 10 is started, the guide mode is set to the water surface mode. Then, a coordinate value Z_{SF} representing a position of the water surface WS is reset, and the guide control to elevate the capsule endoscope 10 to the water surface WS is performed. At this time, as illustrated in FIG. 16, in place of the water bottom mark m13 illustrated in FIG. 6, a water surface mark m16 indicating the position of the water surface WS is displayed at a position on the scale m10 corresponding to the coordinate value Z_{SF} of the water surface WS in the positional information display area m6.

As the coordinate value Z_{SF} of the water surface WS, a maximum value Zₘₐₓ of the coordinate value Z_{CP} of the capsule endoscope 10 after the reset of the coordinate value Z_{SF} is set. Alternatively, as the coordinate value Z_{SF} of the water surface WS, the coordinate value Z_{CP} of the capsule endoscope 10 obtained when the water surface mode is changed to the underwater mode after the capsule endoscope 10 reaches the water surface WS may be set.

After the capsule endoscope 10 reaches the water surface WS, when the capsule endoscope 10 is sunk underwater and subjected to the guide control, a user visually recognizes that the capsule current position mark m14 is separated from the water surface mark m16 in the positional information display area m6, whereby the user can easily grasp that the capsule endoscope 10 has sunk from the water surface WS.

In addition, as a further variation, similar display control can be performed when the capsule endoscope 10 is brought into contact with a lateral inner wall of the organ ST, and then capsule endoscope 10 is separated from the inner wall and caused to float underwater. In this case, the display unit 23 only needs to display a mark (side surface mark) representing a position of the lateral inner wall kept in contact with the capsule endoscope 10 and the capsule current position mark m14 representing the position of the capsule endoscope 10. The user visually recognizes a positional relation between the side surface mark and the capsule current position mark m14, whereby the user can easily grasp whether the capsule endoscope 10 is separated from the lateral inner wall and floats underwater.

### (Fifth Variation)

Next, a fifth variation of the above-mentioned first and second embodiments will be described.

In the above-mentioned first and second embodiments, the magnetic field generating unit 25 that generates a magnetic field to be applied to the permanent magnet 18 provided inside the capsule endoscope 10 is used as a guide unit for the capsule endoscope 10. However, a guide method for the capsule endoscope is not limited to the method using a magnetic field. For example, the capsule endoscope may be guided in such a manner that a propeller is provided in the capsule endoscope and thrust force of the propeller is controlled. Alternatively, the capsule endoscope may be guided in such a manner that an ultrasound motor is provided in the capsule endoscope and drive force of the ultrasound motor is controlled.

The above-mentioned first and second embodiments and first to fifth variations of these embodiments are merely examples for performing the present invention, and the present invention is not limited to these embodiments and variations. In the present invention, a plurality of components disclosed in the first and second embodiments and each of the first to fifth variations can be appropriately combined so as to form various inventions. It is obvious from the above description that the present invention can be variously modified according to a specification or the like, and can further include various other embodiments within a scope of the present invention. Reference Signs List
- 1, 1A: CAPSULE MEDICAL DEVICE GUIDE SYSTEM
- 10: CAPSULE ENDOSCOPE
- 11A, 11B: IMAGING UNIT
- 12A, 12B: ILLUMINATION UNIT
- 13A, 13B: OPTICAL SYSTEM
- 14A, 14B: IMAGE SENSOR
- 15: CONTROL UNIT
- 16: WIRELESS COMMUNICATION UNIT
- 16a: ANTENNA
- 17: POWER UNIT
- 18: PERMANENT MAGNET
- 20, 20A: GUIDE DEVICE
- 20a: BED
- 21: RECEIVING UNIT
- 21a: RECEIVING ANTENNA
- 22: POSITION DETECTING UNIT
- 23: DISPLAY UNIT
- 24: OPERATION INPUT UNIT
- 25: MAGNETIC FIELD GENERATING UNIT
- 26: CONTROL UNIT
- 261: GUIDE CONTROL UNIT
- 262: DISPLAY CONTROL UNIT
- 263: DETERMINATION UNIT
- 27: STORAGE UNIT
- 31, 32: JOYSTICK
- 34U: UP BUTTON
- 34B: DOWN BUTTON
- 35: CAPTURE BUTTON
- 36: APPROACH BUTTON
- 100: CAPSULE-SHAPED CASING
- 101: TUBULAR CASING
- 102, 103: DOME-SHAPED CASING

## Claims

1. A capsule medical device guide system configured to guide a capsule medical device that captures a subject within the subject into which a liquid is introduced, the capsule medical device guide system comprising:
a guide unit configured to guide the capsule medical device;
a position detecting unit configured to detect a position of the capsule medical device to output positional information;
a display unit configured to display the position of the capsule medical device within the subject; and
a control unit configured to cause, based on the positional information, the display unit to display the position of the capsule medical device within the subject and a boundary position of a range in which the capsule medical device is capable of being guided within the subject.

2. The capsule medical device guide system according to claim 1, wherein
the control unit sets a maximum value or a minimum value of the position of the capsule medical device as the boundary position.

3. The capsule medical device guide system according to claim 2, further comprising an operation input unit configured to input, to the control unit, a signal that depends on operation from the outside, wherein
the control unit resets the boundary position in accordance with the signal input from the operation input unit.

4. The capsule medical device guide system according to claim 2, wherein
either a first guide mode in which the capsule medical device is guided while in contact with a boundary of the area or a second guide mode in which the capsule medical device is guided while being separated from the boundary of the area and floating in the liquid is capable of being set switchably, and
the control unit sets, as the boundary position, a maximum value or a minimum value of a position reached by the capsule medical device while the first guide mode is set.

5. The capsule medical device guide system according to claim 2, wherein
the first guide mode is a guide mode in which the capsule medical device is guided while in contact with a boundary of the area on a lower side in a vertical direction, and
the control unit sets, as the boundary position, a minimum value of the position of the capsule medical device in the vertical direction in which an upward orientation is assumed to be positive.

6. The capsule medical device guide system according to claim 2, wherein
the first guide mode is a guide mode in which the capsule medical device is guided while in contact with a boundary of the area on an upper side in a vertical direction, and
the control unit sets, as the boundary position, a maximum value of the position of the capsule medical device in the vertical direction in which an upward orientation is assumed to be positive.

7. The capsule medical device guide system according to claim 1, further comprising an operation input unit configured to input, to the control unit, a signal that depends on operation from the outside, wherein
the control unit sets, as the boundary position, the position of the capsule medical device at a point of time when predetermined operation input is performed on the operation input unit.

8. The capsule medical device guide system according to claim 7, wherein
the control unit resets the boundary position in accordance with the signal input from the operation input unit.

9. The capsule medical device guide system according to claim 7, wherein
either a first guide mode in which the capsule medical device is guided in such a direction as to bring the capsule medical device into contact with a boundary of the area or a second guide mode in which the capsule medical device is guided in such a direction as to cause the capsule medical device to be separated from the boundary of the area and float in the liquid is capable of being set switchably, and
the control unit sets, as the boundary position, the position of the capsule medical device at a point of time when the first guide mode is switched to the second guide mode.

10. The capsule medical device guide system according to claim 4 or 9, wherein
the control unit is configured to:
cause the display unit to display a scale representing the position of the capsule medical device in a predetermined direction within the subject, and to display, on the scale, a first indicator representing the boundary position and a second indicator representing a current position of the capsule medical device,
when the first guide mode is set, cause positions of the first and second indicators to coincide, and cause the first and second indicators to move on the scale in accordance with the position of the capsule medical device, and
when the second guide mode is set, fix the position of the first indicator to the scale, and cause only the second indicator to move on the scale in accordance with the position of the capsule medical device.

11. The capsule medical device guide system according to claim 1, further comprising a determination unit configured to determine, based on a difference between the position of the capsule medical device and the boundary position, a contact state of the capsule medical device with respect to a boundary of the area, wherein
the control unit causes the display unit to display a determination result produced by the determination unit.
